(19) Europäisches Patentamt
European Patent Office
Office européen des brevets

(11) EP 2 851 362 B1

(12) **EUROPEAN PATENT SPECIFICATION**

(45) Date of publication and mention of the grant of the patent:
**27.11.2019 Bulletin 2019/48**

(21) Application number: **13185032.3**

(22) Date of filing: **18.09.2013**

(51) Int Cl.:
*C07C 303/24* (2006.01) *C07C 303/28* (2006.01)
*C07C 305/06* (2006.01) *C07C 305/08* (2006.01)
*C07C 305/20* (2006.01) *C07C 305/24* (2006.01)
*C07C 309/73* (2006.01)

(54) **A method for the production of sulfate or sulfonate esters**

Verfahren zur Herstellung von Sulfat oder Sulfonatestern

Procédé pour la production d'esters de sulfate ou de sulfonate

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.03.2015 Bulletin 2015/13**

(73) Proprietor: **Ulusal Bor Arastirma Enstitusu
06520 Ankara (TR)**

(72) Inventors:
- **Bicak, Niyazi
34469 Istanbul (TR)**
- **Gunes, Deniz
34744 Bostanci
Istanbul (TR)**
- **Akbas, Cem Emre
Bahcelievler
Istanbul (TR)**
- **Sirkecioglu, Okan
34469 Sariyer
Istanbul (TR)**

(74) Representative: **Dericioglu Kurt, Ekin
Ankara Patent Bureau Limited
Kavaklidere Mahallesi
Bestekar Caddesi No: 10
06680 Cankaya, Ankara (TR)**

(56) References cited:
- **DENIZ GUNES ET AL: "ALIPHATIC THIOETHERS BY S-ALKYLATION OF THIOLS VIA TRIALKYL BORATES", PHOSPHORUS, SULFUR AND SILICON AND THE RELATED ELEMENTS, TAYLOR & FRANCIS INC, US, vol. 185, no. 8, 1 January 2010 (2010-01-01), pages 1685-1690, XP008165903, ISSN: 1042-6507, DOI: 10.1080/10426500903213563 [retrieved on 2010-08-02]**
- **OKI ET AL: "Solvothermal synthesis of carbon nanotube-B2O3 nanocomposite using tributyl borate as boron oxide source", INORGANIC CHEMISTRY COMMUNICATIONS, ELSEVIER, AMSTERDAM, NL, vol. 11, no. 3, 31 December 2007 (2007-12-31), pages 275-278, XP022499511, ISSN: 1387-7003, DOI: 10.1016/J.INOCHE.2007.12.015**
- **ITAMAR M MALKOWSKY ET AL: "Facile and Reliable Synthesis of Tetraphenoxybor ates and Their Properties", EUROPEAN JOURNAL OF INORGANIC CHEMISTRY, WILEY-VCH VERLAG, WEINHEIM, DE, vol. 2006, no. 8, 1 March 2006 (2006-03-01), pages 1690-1697, XP008160248, ISSN: 1434-1948, DOI: 10.1002/EJIC.200600074**
- **ANDREI Y. BOCHKOV ET AL: "Synthesis of 3-(5-Methylthiophen-2-yl)coumarins and Their Photochromic Dihetarylethene Derivatives", JOURNAL OF HETEROCYCLIC CHEMISTRY, vol. 50, no. 4, 2 July 2013 (2013-07-02), pages 891-898, XP055089480, ISSN: 0022-152X, DOI: 10.1002/jhet.931**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 851 362 B1

- Alex C. Bissember ET AL: "A Mild, Palladium-Catalyzed Method for the Dehydrohalogenation of Alkyl Bromides: Synthetic and Mechanistic Studies", Journal of the American Chemical Society, vol. 134, no. 34, 20 August 2012 (2012-08-20), pages 14232-14237, XP055493627, US ISSN: 0002-7863, DOI: 10.1021/ja306323x
- Alex C. Bissember ET AL: "A Mild, Palladium-Catalyzed Method for the Dehydrohalogenation of Alkyl Bromides: Synthetic and Mechanistic Studies - Supporting information", Journal of the American Chemical Society, vol. 134, no. 34, 29 August 2012 (2012-08-29), pages 14232-14237, XP055493629, US ISSN: 0002-7863, DOI: 10.1021/ja306323x
- DREGER E E ET AL: "SODIUM ALCOHOL SULFATES PROPERTIES INVOLVING SURFACE ACTIVITY", INDUSTRIAL AND ENGINEERING CHEMISTRY, AMERICAN CHEMICAL SOCIETY, US, vol. 36, no. 7, 1 January 1944 (1944-01-01), pages 610-617, XP001041414, DOI: 10.1021/IE50415A004
- Ko Yasumoto: "Isolation of new aliphatic sulfates and sulfamates as the Daphnia Kairomones inducing morphological change of a phytoplankton Scenedesmus gutwinskii", Chem. Pharm. Bull., 1 January 2008 (2008-01-01), pages 133-136, XP055494545, Retrieved from the Internet: URL:https://www.jstage.jst.go.jp/article/cpb/56/1/56_1_133/_pdf/-char/en [retrieved on 2018-07-23]

## Description

### Field of the Invention

The present invention relates to a method for the production of sulfate or sulfonate esters based on the acidolysis of the boron esters acquired from alcohol and boric acid with sulfuric acid or sulfonic acid.

### Background of the Invention

Sulfate and sulfuric acid esters known as well alkylating agents are used in alkylation of various compounds such as amines, carboxylic acids, alcohol and thiol. Today, alkylating reactions with these compounds are the subject of many researches.

Sodium and potassium salts of sulfuric acid monoesters of long chain alcohols have extensive use in detergent formulations as surface active compounds. Recent searches have shown that sulfate monoesters are part of a number of biological processes such as hormone regulation and molecular recognition.

In order to prepare dialkyl sulfates, methods wherein the alcohols are reacted with tertiary amine-sulfur trioxide complexes are used. Dimethyl sulfate is obtained with the reaction based on direction interaction of sulfuric acid with methanol in high yields. However the said method causes low yields when the other alcohols are used. Yielding a number of by products such as alkene, isoalkene, ether and sulfate ester isomers after the reaction causes this situation.

Besides, industrial or laboratory scale production of alkyl sulfates seems to be confined to the use of tertiary amine- $SO_3$ complexes. International Patent document no WO03020735, an application known in the state of the art, discloses alkylation of saccharides with pyridine-$SO_3$ complex in dimethyl formamide (DMF) or dimethyl sulfoxide (DMSO).

International Patent document no WO9100852, another application known in the state of the art, discloses a preparative method for large-scale production of trialkylamine-$SO_3$ complex by reaction of chlorosulfonic acid with tertiary amines and their use in preparing alkyl sulfates.

As it can be understood from the said documents, early studies realized for sulfation and sulfonation are limited to $SO_3$ and its complexes. Furthermore, using sulfur dioxide ($SO_3$) and its complexes which is highly corrosive and humectant by its nature requires special precautions.

European Patent document no EP1997869, another application known in the state of the art, discloses acquiring borated earth alkaline metal sulfonates with reaction of compounds including boric acid and boric acid anhydride or similar boron compounds with alcohols. In the said process, it is disclosed that materials involving boron such as boric acid, boron anhydride, boron ester and the like can be used as boron source. Borated sulfonic acid is used as starting material.

United States Patent document no US8269038 discloses esterification of sulfonic acids by reaction with low molecular weight dialkyl sulfates in the presence of pyridine in aprotic solvents such as dichloromethane. In another variant of this process, alkyl sulfonates are produced by reaction of alkyl iodides with sulfonic acids using pyridine as acid scavenger.

United States Patent document no US7977525 discloses a process for separating olefins from n-alkanes or their mixtures with iso-alkanes by forming sulfate esters. In this process n-alkanes in upper organic phase can be separated easily from aqueous sulfuric acid phase, but isolation and purification of the sulfate ester has not been described. Obviously the procedure given in this patent has been targeted mainly to remove alkenes from alkane-alkene mixtures.

United States Patent document no US7863479, another application known in the state of the art, discloses sulfation of alcoxylated alcohols by direct action of chlorosulfonic acid or sulfur trioxide complex The resulting monosulfate ester of this process is then neutralized with alkaline hydroxides or amines to give are laundry detergents.

In a related patent, EP 1354872 A1, a process is described for synthesis of tertiary amine salts of sulfate monoesters. The amine components having amide, ester or carbonate units have been claimed to provide softening action in washing formulations. The patent, WO 00/58428 discloses heat-activatable cleaning formulation comprising ethoxylated alkyl ether sulfates. None of those procedures involves the use of sulfuric acid for direct sulfation of alcohols.

However, in a recent patent application (US 2011/0213081 A1), direct action of sulfuric acid has been claimed useful for partial sulfating of ethoxylated polyamines. In this process the water eliminated is removed by continuous vacuuming at 60-85 °C. Conversion yields of this interesting sulfation process can reach 25-30 % within 6-8 h, in terms of the available hydroxyl end groups.

US patent issued in 2002 (US 6,452,035) discloses a trans-sulfation process to produce sulfate esters from alcohols and alcoxylated amines and ethoxylated alcohols. In this process tertiary amine salts of low molecular weight dialkyl sulfates (with $C_1$-$C_4$) are interacted with higher alcohols and residual amines are removed by addition of equivalent sulfuric acid.

In a recent paper and given with reference number [1] titled "Aliphatic Thioethers by S-Alkylation of Thiols via Trialkyl Borates", it has been reported that trialkyl borates can be used for alkylation of thiols to synthesize aliphatic thioethers in the presence of sulfuric acid and pyridine. In the said paper, dialkyl sulfate has been demonstrated as an intermediate product that is consumed in the following reaction as soon as it is formed. Presence of dialkyl sulfate in the mixture has been confirmed by extracting from the aliquot taken from the mixture using ether, but it is not stated that dialkyl sulfates are isolated and purified. The presence of this intermediate product has been proven with H-NMR spectrum of the tiny quantity of the liquid transferred into ether. 100°C is used as the temperature of the process, however dialkyl sulfate formed as intermediate product in long term reactions at 100°C does not remain stable and deteriorates, moreover this process is not applicable at temperatures other than this said temperature. Even though this process shows the production of unstable dialkyl sulfate as intermediate product, it does not involve the production of monoalkyl sulfate esters. The preparation of alkyl tosylates is disclosed in J. Amer. Chem. Soc. (2012), vol. 134 pages 14232-14237 and the preparation of sodium alkyl sulfates is disclosed in Ind. Eng. Chem (1944), vol. 36, pages 610-617.

## Summary of the Invention

The objective of the present invention is to provide a method for the production of sulfate or sulfonate esters based on the acidolysis of boron esters of the alcohols or ethoxylated alcohol, amine and phenols.

Another objective of the present invention is to provide a method which enables safe, cheap and large-scale production of anionic surface active agents comprising alkali metal salts of monoalkyl sulfates in different chain lengths.

Disclosed is also a method for the production of dialkyl sulfate esters which does not involve conventional processes wherein the toxic chemicals such as sulfur trioxide and the like are present.

Yet another objective of the present invention is to provide a method for the production of sulfate or sulfonate esters which does not involve isomeric by product.

## Detailed Description of the Invention

A method for the production of sulfate or sulfonate esters developed to fulfill the objective of the present invention is illustrated in the accompanying figure, in which;

Figure 1 is the flowchart of the method for the production of sulfate or sulfonate esters.

A method for the production of mono alkyl sulfate esters or sulfonated esters of alkali metal salts (10) developed to fulfill the objective of the present invention comprises the steps of

- synthesis of boron ester by the methods given in open literature (11),
    - mixing boric acid and the alcohol (111),
    - heating the mixture (112),
    - adding toluene (113),
    - removing the water (114),
    - removing the toluene (115),
    - obtaining of boron ester (116),

following these steps;

- an acidolysis method for production mono alkyl sulfates of alkali metal salts by reacting boron esters with sulfuric acid; or an acidolysis method for producing alkyl sulfonate esters of alcohols by reacting boron esters with sulfonic acid in high yields (12),
    - adding sulfuric acid or sulfonic acid to the boron ester in medium with solvent wherein dichloroethane is used as solvent, or without solvent (121),
    - stirring the mixture that is prepared (122),
    - removing boric acid which precipitates (123),
    - removing the solvent in case it is used (124),
    - obtaining sulfate or sulfonate esters (125).

In the inventive method for the production of the sulfate and sulfonate esters (10); first the boron esters are synthesized (11). With this purpose, a mixture is prepared by mixing 1 mol boric acid with 3 mol alcohol (111). Then the said mixture is heated at 130°C (112). The heating process (112) is continued until water release is in stoichiometric ratio. This ratio is as 3moles of water release per 1 mol boric acid. Heating process (112) is followed by adding toluene

(113) to the heated mixture. The purpose of adding toluene (113) is to accelerate the progress of the reaction, thus the water release. Water release is completed approximately in 2 hours. Water which comes out as by product as a result of the reaction is removed azeotropically (114) and collected in Dean Stark apparatus. And the toluene which is added to the medium later is removed in vacuo from the medium (115) after the reaction is completed. Therefore, boron ester is obtained as almost colorless liquid having purity over 95% (116). In the first mixture, in case alcohols with less than 8 carbons are used as alcohol, the purity of the yielded boron ester is high. The degree of purity can be measured with H-NMR spectrum after isolating the boron ester by distilling from the reaction mixture under atmospheric pressure or in vacuum and after removing the toluene.

After the synthesis of boron ester (11), sulfate or sulfonate esters are synthesized (12).

In one embodiment disclosed, in order to synthesize the dialkyl sulfate ester (not part of the invention), first sulfuric acid is added to the boron ester in a medium with dichloroethane solvent or without solvent in equivalent ratio (121). This ratio is 1.2-1.6 mol sulfuric acid per 1 mol of boron ester. Sulfuric acid is added dropwise while the boron ester is stirred vigorously at 0°C. In order to increase the dialkyl sulfate yield, 0.5-1.5 mol diphosphopenta oxide or polyphosphoric acid is used per 1 mol of sulfuric acid. After sulfuric acid is added (121), the mixture is continued to be stirred at room temperature for 2-3 hours, and then at 60-90 °C for 24 hours (122). The boric acid deposited as a result of the stirring (122) process is removed from the medium by using a sintered glass filter or centrifuge (123). After removal of the precipitated boric acid (123) and removing the solvent (124), if it is present, dialkyl sulfate is obtained as liquid in 91-99% purity (125). The color of the dialkyl sulfate products which are obtained can be removed with active carbon or similar suitable decolorant agents.

The reaction realized in the said embodiment is as follows: $(R\text{-}O)_3\text{-}B + 3/2\ H_2SO_4 \rightarrow H_3BO_3 + 3/2\ R\text{-}O\text{-}SO_2\text{-}O\text{-}R$ Boron ester Sulfuric acid Boric acid Dialkyl sulfate Here the group R shows ($C_1$-$C_{22}$) alkyl group having 1-22 carbons. In one embodiment of the invention, group R comprises ethereal, aromatic, tertiary amine and amide groups as well as hydrocarbons in its structure. In step of mixing boric acid with alcohol (111), since sulfate ester obtained by using alcohols such as cyclohexanol, dodecanol, cetyl alcohol and octadecanol is a solid melting at low temperatures, solvents such as $CCl_4$ and dichloroethane are required to be used as solvent in step of adding sulfuric acid to the boron ester (121). In this case, the solvent is vaporized with rotary evaporator after the products are filtered through a silica gel column (neutral, 10mm X 100mm). Since the boron esters of the benzylic alcohols are polymerized with the sulfuric acid effect, sulfate esters of the said alcohols cannot be acquired by this way [2].

In one embodiment of the invention, in order to synthesize the monoalkyl sulfate ester, first sulfuric acid is added to the boron ester in the presence of dichloroethane solvent or in a medium without solvent in equivalent ratio (121). This ratio is 2.8-3.5 mol sulfuric acid per 1 mol of boron ester. Sulfuric acid is added dropwise while the boron ester is stirred vigorously at 0°C. After sulfuric acid is added (121), the mixture is stirred at room temperature for 2-3 hours, and then at 60-90 °C for 24 hours (122). The boric acid precipitated as a result of the stirring (122) process is removed from the medium by using a sintered glass filter or a centrifuge (123). After filtering the boric acid (123) and removing the solvent (124) if it is present, the residual monoester is neutralized with 50% NaOH solution at 0°C at the same time being stirred vigorously with a mechanical mixer. This process is performed with 1 mol NaOH solution per 1 mol sulfuric acid. As a result of neutralization, monoalkyl sulfate salt is obtained in purities over 98% (125).

The reactions realized in the said embodiment of the invention are as follows:

$$(R\text{-}O)_3\text{-}B \quad + \quad 3\ H_2SO_4 \quad \rightarrow \quad H_3BO_3 \quad + \quad 3\ R\text{-}O\text{-}SO_2\text{-}OH$$

Boron ester — Sulfuric acid — Boric acid — Sulfate monoester

$$R\text{-}O\text{-}SO_2\text{-}OH \quad + \quad NaOH \quad \rightarrow \quad R\text{-}O\text{-}SO_2\text{-}ONa$$

Sulfate monoester — Sodium hydroxide — Monoalkyl sulfate sodium salt

Here the group R represent ($C_1$-$C_{22}$) alkyl group having 1-22 carbons. The salts of monoalkyl sulfate esters derived from higher alcohols with ($C_6$-$C_{22}$) can be obtained in high purities such as 95% by re-crystallization from ethanol. The monoalkyl sulfates which are obtained comprise the alkyl groups of primary and secondary alcohols and ethoxylated phenol, ethoxylated amine and ethoxylated amino alcohols in various chain lengths including 1-22 carbons.

In another embodiment of the invention, in order to synthesize the monoalkyl sulfonate ester, first sulfonic acid is added to the boron ester in the presence of dichloroethane solvent or in a medium without solvent in equivalent ratio (121). This ratio is 2.8-3.5 mol sulfonic acid per 1 mol of boron ester. Every compound comprising ($-SO_3H$) function can

be used as sulfonic acid. In the preferred embodiment of the invention, benzene sulfonic acid, methane sulfonic acid and/or p-toluene sulfonic acid are used for this purpose. Sulfonic acid is added dropwise while the boron ester is stirred vigorously at 80-100°C (121). After the addition of sulfonic acid, the mixture is stirred for 2-3 hours at 100 °C (122). The boric acid precipitated as a result of the stirring (122) process is removed by using a sintered glass filter or a centrifuge (123). After the solvent is also removed, if present (124), sulfonate ester is obtained in over 90% yields (125).

The reactions carried out in the said embodiment of the invention are as follow and R and $R_1$ represent ($C_1$-$C_{22}$) alkyl groups having 1-22 carbons.

$$(R\text{-}O)_3\text{-}B \quad + \quad 3\ R_1\text{-}SO_3H \quad \rightarrow \quad H_3BO_3 \quad + \quad 3\ R1\text{-}SO_2\text{-}O\text{-}R$$

Boron ester    Sulfonic acid    Boric acid    Sulfonate ester

Sulfate or sulfonate products obtained with the developed method do not involve any by product originating from isomerization. This means sulfate or sulfonate isomers are not present in the obtained products. For example, the product derived from n-butanol is n-butylsulfate, it does not isodibutyl sulfate even in small quantities.

The inventive "method for the production of sulfate or sulfonate esters" are described in details with the following examples.

Example 1 (not part of the invention)

123,6g boric acid, 444g butanol and 250mL toluene is added to a 1000ml volume of glass reactor equipped with reflux condenser and a Dean-Stark trap. The reaction mixture is heated at 130°C and stirred until stoichiometrical amount of water (108mL) is collected in the trap (2-3h). As a result of the reaction, the residual toluene is removed by the suction from the top of the reflux condenser (2.67-3.33 kPa (20-25 mm Hg)). Tributyl borate, boron ester of butyl alcohol, is left as colorless liquid in the reactor. While tributyl borate in the reactor transferred into an ice-bathe is being stirred with a mechanical mixer, 300 g $H_2SO_4$ is added dropwise. After the mixture is stirred in ice-bathe for 1-3 hours, and at room temperature for 2-3 hours, it is stirred at 60-90 °C for 24 hours. In order to eliminate the acid residues, the liquid mixture is mixed with 50g commercial poly(4-vinyl pyridine) and filtered through a silica column (10cm). The light brown liquid product, dibutyl sulfate, is stored in a tightly sealed glass bottle.

Example 2 (not part of the invention)

61.8g boric acid, 300 g cyclohexanol and 250mL toluene is added to a 1000ml volume of glass reactor equipped with reflux condenser Dean-Stark trap. The reaction mixture is heated at 130°C and stirred until stoichiometrical amount of water (54 mL) is collected in the trap (2-3h). As a result of the reaction, the residual toluene is removed by the suction from the top of the reflux condenser (2.67-3.33 kPa (20-25 mm Hg)). As a result of this, tricyclohexyl remains in the reactor, and the boron ester solidifies upon cooling. The solid boron ester dissolves in 300mL dichloroethane and a homogenous mixture is acquired. While tricyclohexyl borate in the reactor transferred into an ice-bathe is being stirred with a mechanical mixer, 150 g $H_2SO_4$(98%) is added dropwise. After the mixture is stirred in ice-bathe for 1-3 hours, and at room temperature for 2-3 hours, it is stirred at 60-90 °C for 24 hours. In order to eliminate the acid residues, the liquid mixture which is cooled is mixed with 30 g commercial poly(4-vinyl pyridine) and filtered through a silica column (10cm). Dichloroethane used as solvent is removed by a rotary evaporator. Dicyclohexyl sulfate is a solid product at room temperature and the product is stored in a glass bottle while it is still hot in order to avoid losses that can occur.

Example 3

30,9g boric acid, 279 g dodecyl alcohol and 250mL toluene is added to a 1000ml volume of glass reactor equipped with reflux condenser Dean-Stark trap. The reaction mixture is heated at 130°C and stirred until stoichiometrical amount of water (27 mL) is collected in the trap (2-3h). As a result of the reaction, the residual toluene is removed by the suction from the top of the reflux condenser (2.67-3.33 kPa (20-25 mm Hg)). As a result of this, dodecyl borate remains in the reactor, and the boron ester solidifies upon cooling. The solid boron ester dissolves in 300mL dichloroethane and a homogenous mixture is acquired. While tridodecyl borate in the reactor transferred into an ice-bathe is being stirred with a mechanical mixer, 150 g $H_2SO_4$(98%) is added dropwise. Precipitated boric acid is filtered through a sintered glass funnel and the filtered liquid is transferred to 2L volume Erlenemeyer flask cooled in ice-bath. Concentrated methanol solution comprising 60g NaOH solution previously prepared is added dropwise for 30 minutes on boron ester stirred vigorously. Precipitation of sodium dodecyl sulfate is observed clearly during this addition. When the product is filtered, milky white obtained solids are washed with cold methanol solution and dried for 3 hours at 50°C under vacuum.

451g product is yielded quantitatively. H-NMR spectrum of the product re-crystallized from ethanol represents exactly the spectrum of the sodium dodecyl sulfate (SDS) in the literature.

Example 4

6.9g boric acid, 132 g ethoxylated nonyl phenol (ethoxylation number is approximately 4) and 250mL toluene is added to a 1000ml volume of glass reactor equipped with reflux condenser Dean-Stark trap. The reaction mixture is heated at 130°C and stirred until stoichiometrical amount of water (6 mL) is collected in the trap (2-3h). As a result of the reaction, the residual toluene is removed by the suction from the top of the reflux condenser (2.67-3.33 kPa (20-25 mm Hg)). A homogenous solution is obtained by dissolving waxy textured product in 200ml dichloroethane. While boron ester in the reactor transferred into an ice-bathe is being stirred with a mechanical mixer, 30 g $H_2SO_4$(98%) is added dropwise. Precipitated boric acid is filtered through a sintered glass funnel and the filtered liquid is transferred to 2L volume Erlenemeyer flask cooled in ice-bath. Methanol solution comprising 12 g NaOH solution previously prepared is added dropwise for 30 minutes on boron ester stirred vigorously. Dichloroethane used as solvent is removed by a rotary evaporator and the product is poured into 300mL acetone. The white product is collected by filtration and washed with cold methanol and dried at 50 °C for 3 hours under vacuum. Purity of the final product is checked and proved by H-NMR taken $D_2O$ solvent.

Example 5

6.19g boric acid, 22.2 g butanol and 50mL toluene is added to a 500ml volume of glass reactor equipped with reflux condenser Dean-Stark trap. The reaction mixture is heated at 130°C and stirred until stoichiometrical amount of water (5.4 mL) is collected in the trap (2-3h). As a result of the reaction, the residual toluene is removed by the suction from the top of the reflux condenser (2.67-3.33 kPa (20-25 mm Hg)).

Tributyl borate, boron ester of the butyl alcohol, remains in the reactor as colorless liquid; this product is transformed into ester of the acid by being reacted with p-toluene sulfonic acid. However, commercial p-toluene sulfonic acid sold as monohydrate salt involves one mol (18mL) water in each mol by its nature; and the water should be removed from the reaction medium. The following method is applied for this:

57g commercial p-toluene sulfonic acid and 50mL cyclohexan is placed in a 250mL volume round bottom flask equipped with Dean-Stark trap with reflux condenser. The reaction mixture is heated under reflux condenser and heated until 5.4mL of water is collected (approximately 1 hour). While the water and cyclohexan is collected in the trap, anhydrous p-toluene sulfonic acid remains in the reaction flask. After the system is cooled, previously prepared 23g tributyl borate is added to the reaction flask together with 50mL dichloroethane; stirred at 100°C for 8 hours and heated. 150mL of cold water is added to the mixture which is taken to the seperatory funnel after cooling, the organic phase, lower phase, is separated and washed with 150mL of sodium hydrogen carbonate solution (3%). The lower organic phase is washed again with 150mL of pure water and a product purified from excess acid and impurities is yielded. The liquid product is filtered after drying with 6 g anhydrous sodium sulfate. Butyl p-toluene sulfonate is yielded (55-58g) by removing dichloroethane used as solvent by rotary evaporator. H-NMR of the product yielded here shows exact match with the product obtained by the reaction of p-toluene sulfonyl chloride with butanol, as described in the literature.

Determination of the sulfate amount included by the monoalkyl sulfates produced in the inventive method for the production of sulfate and sulfonate esters is identified by precipitating sulfate groups in hydrous solution as $Ba_2SO_4$. 1g of sample is mixed with 10% 20mL NaOH solution and heated for 1 hour under the reflux condenser. After the solution is cooled, 1.5g $BaCl_2$ solution dissolved in 10mL pure water is added to mixture and heated again for 2 hours. White $BaSO_4$ which is formed is filtered, washed with 2X20mL distilled water and dried at 80°C under vacuum for 4 hours.

The percentage of the sulfate is calculated with the following formula:

$$\text{Sulfate }\%\,(\text{w/w}) = \frac{96\ \text{m}}{233.32\ \text{m}_0} \times 100$$

wherein 233.32 and 96 are the molecule weights of barium sulfate ($BaSO_4$) and sulfate ions respectively; and $m_o$ and m denote weights of the sample and $BaSO_4$ respectively.

Within the framework of these basic concepts, it is possible to develop various embodiments of the inventive "a method for the production of sulfate or sulfonate esters". The invention cannot be limited to the examples described herein and it is as defined in the claims.

## REFERENCES

[1] Gunes D, Sirkecioglu O, Bicak N. Aliphatic Thioethers by S-Alkylation of Thiols via Trialkyl Borates. Phosphorus Sulfur and Silicon and The Related Elements. 2010, 185(8): 1685-1690.
[2] Gunes D., Yagci Y., Bicak N. Synthesis of soluble poly(p-phenylene methylene) from tribenzylborate by acid-catalyzed polymerization. Macromolecules. 2010;43(19):7993-7997

## Claims

**1.** A method for the production of monoalkyl sulfate esters of alkali metal salts or sulfonate esters (10) **comprising** the steps of

- synthesis of boron esters by the methods given in open literature (11),
    - mixing boric acid and the alcohol (111),
    - heating the mixture (112),
    - adding toluene (113),
    - removing the water (114),
    - removing the toluene (115),
    - obtaining of boron ester (116), and **characterized by** the steps of
- an acidolysis method for production of mono alkyl sulfates of alkali metal salts by reacting boron esters with sulfuric acid; or an acidolysis method for producing alkyl sulfonate esters of alcohols by reacting boron esters with sulfonic acid in high yields (12),
    - adding sulfuric acid or sulfonic acid to the boron ester in medium with solvent wherein dichloroethane is used as solvent, or without solvent (121),
    - stirring the mixture that is prepared (122),
    - removing boric acid which precipitates (123),
    - removing the solvent in case it is used (124),
    - obtaining sulfate or sulfonate esters (125)

wherein in the monoalkyl sulfate esters of alkali metal salts which are obtained "alkyl" represents a group having 1-22 carbons and comprises alkyl groups of primary and secondary alcohols and ethoxylated phenol, ethoxylated amine and ethoxylated amine alcohols; or in the sulfonate esters "alkyl" represents a (C1-C22) alkyl group having 1-22 carbons.

**2.** A method for the production of monoalkyl sulfate esters of alkali metal salts (10) according to claim 1, **characterized by** the step of adding sulfuric acid to boron ester (121), wherein 2.8-3.5 mol sulfuric acid is added for 1 mol of boron ester in a medium with or without solvent.

**3.** A method for the production of monoalkyl sulfate esters of alkali metal salts (10) according to claim 2, **characterized by** the step of adding sulfuric acid to boron ester (121), wherein sulfuric acid is added dropwise while boron ester is stirred vigorously at 0 °C in a medium with or without solvent.

**4.** A method for the production of mono alkyl sulfate esters of alkali metal salts (10) according to claims 2-3, **characterized by** the step of stirring the mixture for 2-3 hours at room temperature and then at 60-90°C for 24 hours (122).

**5.** A method for the production of mono alkyl sulfate esters of alkali metal salts (10) according to according to any one of the claims 2-4, **characterized by** the step of removing boric acid (123) wherein the precipitated boric acid as a result of the stirring process (122) is removed from the medium by a sintered glass filter or a centrifuge.

**6.** A method for the production of monoalkyl sulfate esters (10) according to according to any one of the claims 2-5, **characterized by** the step of obtaining sulfate (125) wherein monoalkyl sulfate esters of alkali metal salts having purity over 95% are yielded by neutralizing with %50 NaOH solution at 0°C adding 1 mol NaOH per 1 mol sulfuric

acid, at the same time by stirring the monoester, remaining after the processes of removing the boric acid (123) and removing the solvent if it is present (124), vigorously with a mechanic mixer.

**7.** A method for the production of mono alkyl sulfate esters of alkali metal salts (10) according to any one of the claim 2-6, **characterized by** the step of synthesizing sulfate esters (125) wherein monoalkyl sulfate is obtained with the following reactions:

$$(R\text{-}O)_3\text{-}B + 3\ H_2SO_4 \rightarrow H_3BO_3 + 3\ R\text{-}O\text{-}SO_2\text{-}OH$$

$$R\text{-}O\text{-}SO_2\text{-}OH + NaOH \rightarrow R\text{-}O\text{-}SO_2\text{-}ONa$$

wherein "alkyl" (R) term (including also the polymeric ones) represents a group having 1-22 carbons and comprises alkyl groups of primary and secondary alcohols and ethoxylated phenol, ethoxylated amine and ethoxylated amine alcohols.

**8.** A method for the production of sulfonate esters (10) according to claim 1, **characterized by** the step of adding sulfonic acid to boron ester (121), wherein 2,8-3,5 mol sulfonic acid is added for 1 mol of boron ester in a medium with or without solvent.

**9.** A method for the production of sulfonate esters (10) according to claim 8, **characterized by** the step of adding sulfonate acid to boron ester (121) wherein sulfonic acid added to boron ester is not limited to benzene sulfonic acid, methane sulfonic acid, p-toluene sulfonic acid, it can also be every compound comprising bisulfide ($-SO_3H$) functional group.

**10.** A method for the production of sulfonate esters (10) according to any one of the claims 8-9, **characterized by** the step of adding sulfonic acid to boron ester dropwise (121), wherein sulfonic acid is added dropwise while boron ester is stirred vigorously at 80-100°C in a medium with or without solvent.

**11.** A method for the production of sulfonate esters (10) according to any one of the claims 8-10, **characterized by** the step of stirring the mixture (122), wherein the mixture which is prepared is stirred for 24 hours at 100°C.

**12.** A method for the production of sulfonate esters (10) according to according to any one of the claims 8-11, **characterized by** the step of obtaining sulfonate (125) wherein sulfonate esters are yielded in purities over 90% after removing precipitated boric acid (123) and removing solvent if it is present (124).

**13.** A method for the production of sulfonate esters (10) according to any one of the claims 8-12, **characterized by** the step of synthesizing sulfonate esters (12) wherein the sulfonate ester is obtained with the following reaction

$$(R\text{-}O)_3\text{-}B + 3\ R_1\text{-}SO_3H \rightarrow H_3BO_3 + 3\ R_1\text{-}SO_2\text{-}O\text{-}R$$

wherein "alkyl" (R) term (including also the polymeric ones) represents (C1-C22) alkyl groups having 1-22 carbons.

**14.** A method for the production of mono alkyl sulfate esters or sulfonate esters of alkali metal salts (10) according to any one of the preceding claims, wherein the produced sulfate and sulfonate products do not involve any intermediate products originating from the isomerization.

## Patentansprüche

**1.** Verfahren zur Herstellung von Monoalkylsulfatestern von Alkalimetallsalzen oder Sulfonatestern (10), **umfassend** die Schritte von

- Synthese von Borestern nach den in der offenen Literatur angegebenen Verfahren (11),
- Mischen von Borsäure und Alkohol (111),
- Erwärmen der Mischung (112),
- Zugabe von Toluol (113),
- Entfernen des Wassers (114),
- Entfernen des Toluols (115),

- Erhalten von Borester (116), und **gekennzeichnet durch** die Schritte von
- ein Acidolyseverfahren zur Herstellung von Monoalkylsulfaten von Alkalimetallsalzen durch Umsetzung von Borestern mit Schwefelsäure; oder ein Acidolyseverfahren zur Herstellung von Alkylsulfonatestern von Alkoholen durch Umsetzung von Borestern mit Sulfonsäure in hoher Ausbeute (12),
- Zugabe von Schwefelsäure oder Sulfonsäure zum Borester im Medium mit Lösungsmittel, wobei dichloroethane is used as solvent, or without solvent (121),
- Rühren der vorbereiteten Mischung (122),
- Entfernen von ausfälliger Borsäure (123),
- Entfernen des Lösungsmittels, falls es verwendet wird (124),
- Erhalten von Sulfat- oder Sulfonatestern (125)

wobei in den Monoalkylsulfatestern von Alkalimetallsalzen, die erhalten werden, "Alkyl" eine Gruppe mit 1-22 Kohlenstoffatomen darstellt und Alkylgruppen von primären und sekundären Alkoholen und ethoxyliertem Phenol umfasst, ethoxyliertes Amin und ethoxylierte Aminalkohole; oder in den Sulfonatestern "Alkyl" eine (C1-C22) Alkylgruppe mit 1-22 Kohlenstoffatomen darstellt.

**2.** Verfahren zur Herstellung von Monoalkylsulfatestern von Alkalimetallsalzen (10) nach Anspruch 1, **gekennzeichnet durch** den Schritt der Zugabe von Schwefelsäure zu Borester (121), wobei 2.8-3.5 Mol Schwefelsäure für 1 Mol Borester in einem Medium mit oder ohne Lösungsmittel zugegeben wird.

**3.** Verfahren zur Herstellung von Monoalkylsulfatestern von Alkalimetallsalzen (10) nach Anspruch 2, **gekennzeichnet durch** den Schritt der Zugabe von Schwefelsäure zu Borester (121), wobei Schwefelsäure tropfenweise zugegeben wird, während Borester in einem Medium mit oder ohne Lösungsmittel bei 0°C stark gerührt wird.

**4.** Verfahren zur Herstellung von Monoalkylsulfatestern von Alkalimetallsalzen (10) nach den Ansprüchen 2-3, **gekennzeichnet durch** den Schritt des Rührens der Mischung für 2-3 Stunden bei Raumtemperatur und dann bei 60-90°C für 24 Stunden (122).

**5.** Verfahren zur Herstellung von Monoalkylsulfatestern von Alkalimetallsalzen (10) nach einem der Ansprüche 2-4, **gekennzeichnet durch** den Schritt des Entfernens von Borsäure (123), wobei die ausgefällte Borsäure als Ergebnis des Rührprozesses (122) durch einen Sinterglasfilter oder eine Zentrifuge aus dem Medium entfernt wird.

**6.** Verfahren zur Herstellung von Monoalkylsulfatestern (10) nach einem der Ansprüche 2-5, **gekennzeichnet durch** den Schritt des Erhaltens von Sulfat (125), wobei Monoalkylsulfatester von Alkalimetallsalzen mit einer Reinheit von über 95% durch Neutralisation mit %50 NaOH-Lösung bei 0°C unter Zugabe von 1 mol NaOH pro 1 mol Schwefelsäure, gleichzeitig durch Rühren des Monoesters, der nach den Verfahren zum Entfernen der Borsäure (123) und Entfernen des Lösungsmittels, wenn es vorhanden ist (124), übrig bleibt, mit einem mechanischen Mischer kraftvoll erzeugt werden.

**7.** Verfahren zur Herstellung von Monoalkylsulfatestern von Alkalimetallsalzen (10) nach einem der Ansprüche 2-6, **gekennzeichnet durch** den Schritt der Synthese von Sulfatestern (125), wobei Monoalkylsulfat mit den folgenden Reaktionen erhalten wird:

$$(R\text{-}O)_3\text{-}B + 3H_2SO_4 \rightarrow H_3BO_3 + 3\ R\text{-}O\text{-}SO_2\text{-}OH$$

$$R\text{-}O\text{-}SO_2\text{-}OH + NaOH \rightarrow R\text{-}O\text{-}SO_2\text{-}ONa$$

wobei der "Alkyl"-Term (R) (einschließlich auch der polymeren) eine Gruppe mit 1-22 Kohlenstoffatomen darstellt und Alkylgruppen von primären und sekundären Alkoholen und ethoxyliertem Phenol, ethoxyliertem Amin und ethoxylierten Aminalkoholen umfasst.

**8.** Verfahren zur Herstellung von Sulfonatestern (10) nach Anspruch 1, **gekennzeichnet durch** den Schritt der Zugabe von Sulfonsäure zu Borester (121), wobei 2,8-3,5 Mol Sulfonsäure für 1 Mol Borester in einem Medium mit oder ohne Lösungsmittel zugegeben wird.

**9.** Verfahren zur Herstellung von Sulfonatestern (10) nach Anspruch 8, **gekennzeichnet durch** den Schritt der Zugabe von Sulfonatsäure zu Borester (121), wobei die dem Borester zugegebene Sulfonsäure nicht auf Benzolsulfonsäure, Methansulfonsäure, p-Toluolsulfonsäure beschränkt ist, es kann auch jede Verbindung sein, die Bisulfid

(-$SO_3H$)-funktionelle Gruppe umfasst.

**10.** Verfahren zur Herstellung von Sulfonatestern (10) nach einem der Ansprüche 8-9, **gekennzeichnet durch** den Schritt der Zugabe von Sulfonsäure zu Borester tropfenweise (121), wobei Sulfonsäure tropfenweise zugegeben wird, während Borester bei 80-100°C in einem Medium mit oder ohne Lösungsmittel stark gerührt wird.

**11.** Verfahren zur Herstellung von Sulfonatestern (10) nach einem der Ansprüche 8-10, **gekennzeichnet durch** den Schritt des Rührens der Mischung (122), wobei die hergestellte Mischung 24 Stunden lang bei 100°C gerührt wird.

**12.** Verfahren zur Herstellung von Sulfonatestern (10) nach einem der Ansprüche 8-11, **gekennzeichnet durch** den Schritt des Erhaltens von Sulfonat (125), wobei Sulfonatester in Reinheiten von mehr als 90% nach dem Entfernen von gefällter Borsäure (123) und dem Entfernen von Lösungsmittel, wenn es vorhanden ist (124), ausgedrückt werden

**13.** Verfahren zur Herstellung von Sulfonatestern (10) nach einem der Ansprüche 8-12, **gekennzeichnet durch** den Schritt der Synthese von Sulfonatestern (12), wobei der Sulfonatester mit der folgenden Reaktion erhalten wird

$$(R\text{-}O)_3\text{-}B + 3R_1\text{-}SO_3H \rightarrow H_3BO_3 + 3\ R_1\text{-}SO_2\text{-}O\text{-}R$$

wobei der "Alkyl"-Term (R) (einschließlich auch der polymeren) (C1-C22) Alkylgruppen mit 1-22 Kohlenstoffatomen darstellt.

**14.** Verfahren zur Herstellung von Monoalkylsulfatestern oder Sulfonatestern von Alkalisalzen (10) nach einem der vorstehenden Ansprüche, wobei die hergestellten Sulfat- und Sulfonatprodukte keine Zwischenprodukte beinhalten, die aus der Isomerisierung stammen.

## Revendications

**1.** Procédé de production d'esters monoalkyl sulfates de sels de métaux alcalins ou d'esters sulfonates (10) **comprenant** les étapes suivantes

- synthèse d'esters de bore par les méthodes décrites dans la littérature ouverte (11),

  - mélange de l'acide borique et de l'alcool (111),
  - chauffage du mélange (112),
  - ajout de toluène (113),
  - élimination de l'eau (114),
  - élimination du toluène (115),
  - obtention d'ester de bore (116), et **caractérisé par** les étapes suivantes

- un procédé d'acidolyse pour la production de monoalkyl sulfates de sels de métaux alcalins par réaction d'esters de bore avec de l'acide sulfurique ; ou un procédé d'acidolyse pour produire des esters alkyl sulfonates d'alcools par réaction d'esters de bore avec de l'acide sulfonique à haut rendement (12),

  - ajout d'acide sulfurique ou d'acide sulfonique à l'ester de bore dans un milieu avec solvant dans lequel le dichloroéthane est utilisé comme solvant, ou sans solvant (121),
  - agitation du mélange préparé (122),
  - élimination de l'acide borique qui précipite (123),
  - élimination du solvant en cas d'utilisation (124),
  - obtention de sulfate ou d'esters sulfonates (125)

dans lequel, dans les esters monoalkyl sulfates de sels de métaux alcalins qui sont obtenus, "alkyle" représente un groupe ayant 1-22 atomes de carbone et comprend des groupes alkyle d'alcools primaires et secondaires et de phénol éthoxylé, d'amine éthoxylée et d'alcools d'amine éthoxylée ; ou dans les esters sulfonates, "alkyle" représente un groupe alkyle en (C1-C22) ayant de 1-22 atomes de carbone.

**2.** Procédé de production d'esters monoalkyl sulfate de sels de métaux alcalins (10) selon la revendication 1, **carac-**

**térisé par** l'étape de l'ajout de l'acide sulfurique à l'ester de bore (121), dans laquelle on ajoute 2.8-3.5 moles d'acide sulfurique pour 1 mole d'ester de bore dans un milieu avec ou sans solvant.

3. Procédé de production d'esters monoalkyl sulfate de sels de métaux alcalins (10) selon la revendication 2, **caractérisé par** l'étape de l'ajout de l'acide sulfurique à un ester de bore (121), dans laquelle de l'acide sulfurique est ajouté goutte à goutte tandis que l'ester de bore est agité vigoureusement à 0 °C dans un milieu avec ou sans solvant.

4. Procédé de production d'esters monoalkyl sulfate de sels de métaux alcalins (10) selon les revendications 2-3, **caractérisé par** l'étape d'agitation le mélange pendant 2-3 heures à température ambiante, puis à 60-90°C pendant 24 heures (122).

5. Procédé de production d'esters monoalkyl sulfate de sels de métaux alcalins (10) selon l'une quelconque des revendications 2-4, **caractérisé par** l'étape d'élimination de l'acide borique (123) dans laquelle l'acide borique précipité résultant du processus de l'agitation (122) est éliminé du milieu par un filtre en verre fritté ou une centrifugeuse.

6. Procédé de production d'esters monoalkyl sulfate (10) selon l'une quelconque des revendications 2-5, **caractérisé par** l'étape d'obtention de sulfate (125) dans laquelle des esters monoalkyl sulfate de sels de métaux alcalins ayant une pureté supérieure à 95 % sont obtenus par neutralisation avec une solution à 50 % de NaOH à 0°C en ajoutant 1 mole de NaOH pour 1 mole d'acide sulfurique, en même temps par agitation du monoester, restant après les processus d'élimination de l'acide borique (123) et d'élimination du solvant s'il est présent (124), vigoureusement avec un mélangeur mécanique.

7. Procédé de production d'esters monoalkyl sulfate de sels de métaux alcalins (10) selon l'une quelconque des revendications 2-6, **caractérisé par** l'étape de synthèse d'esters sulfates (125) dans laquelle le monoalkyl sulfate est obtenu avec les réactions suivantes :

$$(R\text{-}O)_3\text{-}B + 3H_2SO_4 \rightarrow H_3BO_3 + 3\ R\text{-}O\text{-}SO_2\text{-}OH$$

$$R\text{-}O\text{-}SO_2\text{-}OH + NaOH \rightarrow R\text{-}O\text{-}SO_2\text{-}ONa$$

dans laquelle le terme "alkyle" (R) (y compris les polymères) représente un groupe ayant de 1-22 atomes de carbone et comprend des groupes alkyle d'alcools primaires et secondaires et de phénol éthoxylé, d'amine éthoxylée et d'alcools d'amine éthoxylée.

8. Procédé de production d'esters sulfonates (10) selon la revendication 1, **caractérisé par** l'étape de l'ajout de l'acide sulfonique à l'ester de bore (121), dans laquelle on ajoute 2.8-3.5 moles d'acide sulfonique pour 1 mole d'ester de bore dans un milieu avec ou sans solvant.

9. Procédé de production d'esters sulfonates (10) selon la revendication 8, **caractérisé par** l'étape de l'ajout d'acide sulfonate à l'ester de bore (121) dans laquelle l'acide sulfonique ajouté à l'ester de bore n'est pas limité à l'acide benzène sulfonique, l'acide méthane sulfonique, l'acide p-toluène sulfonique, il peut également s'agir de tout composé comprenant un groupe fonctionnel bisulfure ($-SO_3H$).

10. Procédé de production d'esters sulfonates (10) selon l'une quelconque des revendications 8-9, **caractérisé par** l'étape de l'ajout d'acide sulfonique à un ester de bore goutte à goutte (121), dans laquelle de l'acide sulfonique est ajouté goutte à goutte tandis que l'ester de bore est agité vigoureusement à 80-100°C dans un milieu avec ou sans solvant.

11. Procédé de production d'esters sulfonates (10) selon l'une quelconque des revendications 8-10, **caractérisé par** l'étape d'agitation du mélange (122), dans laquelle le mélange qui est préparé est agité pendant 24 heures à 100°C.

12. Procédé de production d'esters sulfonates (10) selon l'une quelconque des revendications 8-11, **caractérisé par** l'étape d'obtention de sulfonate (125) dans laquelle des esters sulfonates sont obtenus dans des puretés supérieures à 90 % après élimination de l'acide borique précipité (123) et élimination du solvant si celui-ci est présent (124).

13. Procédé de production d'esters sulfonates (10) selon l'une quelconque des revendications 8-12, **caractérisé par** l'étape de synthèse d'esters sulfonates (12) dans laquelle les esters sulfonates sont obtenus par la réaction suivante

$$(R\text{-}O)_3\text{-}B + 3R_1\text{-}SO_3H \rightarrow H_3BO_3 + 3\ R_1\text{-}SO_2\text{-}O\text{-}R$$

dans laquelle le terme "alkyle" (R) (y compris aussi les polymères) représente des groupes alkyle en (C1-C22) ayant 1-22 atomes de carbone.

**14.** Procédé de production d'esters monoalkyl sulfates ou d'esters sulfonates de sels de métaux alcalins (10) selon l'une quelconque des revendications précédentes, dans lequel les produits de sulfate et de sulfonate produits n'impliquent aucun produit intermédiaire provenant de l'isomérisation.

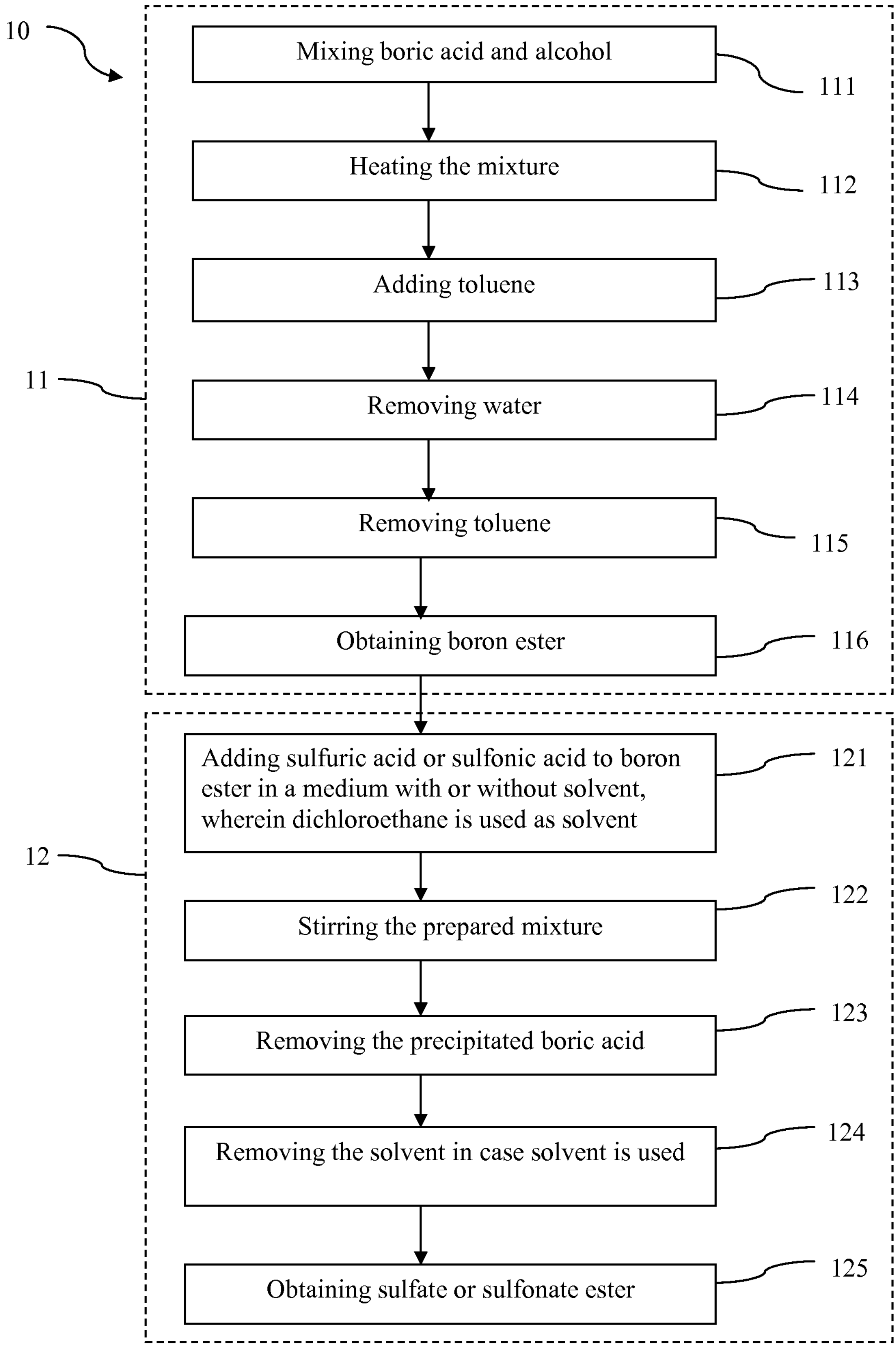
10
11
Mixing boric acid and alcohol
111
Heating the mixture
112
Adding toluene
113
Removing water
114
Removing toluene
115
Obtaining boron ester
116
12
Adding sulfuric acid or sulfonic acid to boron ester in a medium with or without solvent, wherein dichloroethane is used as solvent
121
Stirring the prepared mixture
122
Removing the precipitated boric acid
123
Removing the solvent in case solvent is used
124
Obtaining sulfate or sulfonate ester
125

**Figure 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03020735 A
- WO 9100852 A
- EP 1997869 A
- US 8269038 B
- US 7977525 B
- US 7863479 B
- EP 1354872 A1
- WO 0058428 A
- US 20110213081 A1
- US 6452035 B


### Non-patent literature cited in the description


- *J. Amer. Chem. Soc.,* 2012, vol. 134, 14232-14237
- *Ind. Eng. Chem,* 1944, vol. 36, 610-617
- **GUNES D ; SIRKECIOGLU O ; BICAK N.** Aliphatic Thioethers by S-Alkylation of Thiols via Trialkyl Borates. *Phosphorus Sulfur and Silicon and The Related Elements,* 2010, vol. 185 (8), 1685-1690
- **GUNES D. ; YAGCI Y. ; BICAK N.** Synthesis of soluble poly(p-phenylene methylene) from tribenzylborate by acid-catalyzed polymerization. *Macromolecules,* 2010, vol. 43 (19), 7993-7997